# EUROPÄISCHE PATENTANMELDUNG

(11) **EP 0 725 049 A1**
(43) Veröffentlichungstag der Anmeldung: **07.08.1996**
(21) Anmeldenummer: 96100679.8
(22) Anmeldetag: 18.01.1996
(51) Int. Cl.: C07B 37/04, C07C 41/30, C07C 45/68, C07C 43/215, C07C 49/255

(54) **Verfahren zur Herstellung von aromatischen Olefinen unter Katalyse von Palladacyclen**

(30) Priorität: 01.02.1995 DE 19503119
(71) Anmelder: HOECHST AKTIENGESELLSCHAFT, 65929 Frankfurt am Main (DE)
(72) Erfinder: Beller, Matthias, Dr., D-65510 Idstein (DE); Tafesh, Ahmed, Dr., D-65779 Kelkheim (DE); Herrmann, Wolfgang A., Prof. Dr., D-85354 Freising (DE)

(57) **Zusammenfassung**

Die Erfindung betrifft ein Verfahren zur Herstellung von monofunktionellen, bi-und polyfunktionellen aromatischen Olefinen der Formel (I) 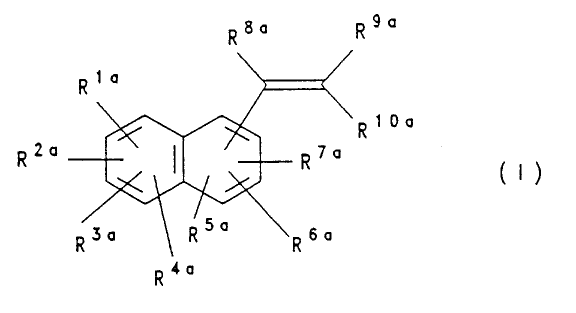
worin
R^{1a} bis R^{7a} unabhängig voneinander Wasserstoff, C₁-C₈-Alkyl, Alkoxy-(C₁-C₈), Acyloxy-(C₁-C₈), OPhenyl, Phenyl, Fluor, Chlor, Brom, Iod, OH, NO₂, OSO₂CF₃, CN, COOH, CHO, SO₃H, SO₂R, SOR, NH₂, NHAlkyl-(C₁-C₈), N-Alkyl₂-(C₁-C₈), CHal₃, NHCO-Alkyl-(C₁-C₄), N-Alkyl-(C₁-C₄)-CO-Alkyl-(C₁-C₄), COO-Alkyl-(C₁-C₈), CONH₂, CO-Alkyl-(C₁-C₈), NHCOH, NCOO-Alkyl-(C₁-C₄), CO-Phenyl, COO-Phenyl, PO-Phenyl₂, PO-Alkyl₂-(C₁-C₄), wobei einer der Reste R^{1a} bis R^{7a} auch 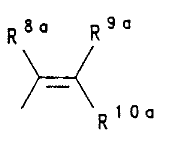
darstellen kann,
R^{8a} Wasserstoff, Alkyl(C₁-C₈), Phenyl, O-Alkyl-(C₁-C₈), Fluor
R^{9a} und R^{10a} unabhängig voneinander Wasserstoff, CN, CO₂H, CO₂-Alkyl-(C₁-C₈), CONH₂, CONH-Alkyl-(C₁-C₄), CON(Alkyl)₂-(C₁-C₄), Fluor, CO₂-Phenyl, Alkyl, (C₁-C₈)-Phenyl, PO(Phenyl), PO(Alkyl-(C₁-C₄))₂, CO-Phenyl, CO-Alkyl-(C₁-C₄), O-Alkyl-(C₁-C₄), NH-Alkyl-(C₁-C₄), PO₃H, SO₃H, SO₃-Alkyl-(C₁-C₄), SO₂-Alkyl-(C₁-C₄), O-Phenyl, bedeuten, durch Umsetzung von Halogenaromaten der allgemeinen Formel (II) 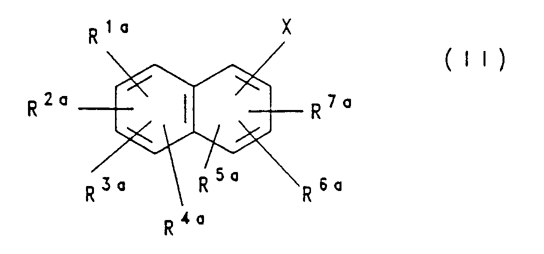
mit Olefinen der allgemeinen Formel (III) 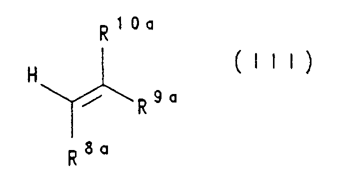
worin R^{1a} bis R^{10a} die angegebene Bedeutung besitzen, wobei einer der Reste R^{1a} bis R^{7a} auch für X stehen kann und X Iod, Brom, Chlor, OSO₂CF₃, OSO₂Phenyl, OSO₂CH₃ bedeutet, dadurch gekennzeichnet, daß man als Katalysator eine Palladiumverbindung der allgemeinen Formel (IV) 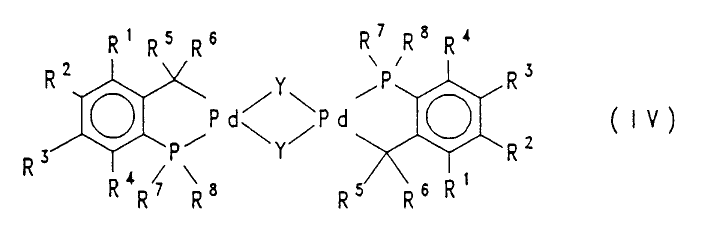
worin R¹, R², R³, R⁴, R⁵, R⁶ unabhängig voneinander Wasserstoff, (C₁-C₄)-Alkyl, (C₁-C₄)-Alkoxy, Fluor, NH₂, NH-Alkyl(C₁-C₄), N(Alkyl)₂-(C₁-C₄), CO₂Alkyl-(C₁-C₄), OCO-Alkyl-(C₁-C₄), oder Phenyl bedeuten, oder R¹ und R², R² und R³, R³ und R⁴, R⁵ und R⁶ zusammen einen aliphatischen oder aromatischen Ring bilden, und
R⁷, R⁸ (C₁-C₈)-Alkyl, (C₃-C₁₂)-Cycloalkyl, substituiertes oder unsubstituiertes Aryl sind
Y ein Anion einer anorganischen oder organischen Säure bedeutet, einsetzt.

## Beschreibung

Die vorliegende Erfindung betrifft ein neues Verfahren zur Herstellung von aromatischen Olefinen mit neuartigen Katalysatoren, sogenannten Palladacyclen.

Aromatische Olefine haben technische Bedeutung als Feinchemikalien, Ausgangsprodukte für Polymere, UV-Absorber und Wirkstoffvorprodukte.

Eine häufig angewandte Methode zur Synthese von aromatischen Olefinen im universitären Bereich ist die Heck-Reaktion, bei der Iod- oder Bromaromaten und in Ausnahmefällen Chloraromaten mit Olefinen in Gegenwart von Palladiumkatalysatoren umgesetzt werden. Übersichten, die diese Methodik beschreiben, findet man bspw. in R.F. Heck, Acc. Chem. Res. 1979, 12, 146; R.F. Heck, Org. React. 1982, 27, 345; R.F. Heck, Palladium Reagents in Synthesis, Academic Press, London 1985.

Katalysatoren, die im Rahmen der Heck-Reaktion verwendet werden, sind Palladiumverbindungen. Obwohl sowohl Palladium(II)- als auch Palladium(O)-Komplexe bei Heck-Reaktionen eingesetzt werden, ist es doch allgemein akzeptiert, daß lediglich Palladium(O)-Verbindungen die eigentlichen Katalysatoren der Reaktion sind. Insbesondere formuliert man in der Literatur koordinativ ungesättigte 14-Elektronen Palladium(O)-Spezies, welche in der Regel mit schwachen Donorliganden wie Phosphanen stabilisiert werden.

Trotz der Vielzahl von Veröffentlichungen auf dem Gebiet der Heck-Reaktion sind bisher keine Beispiele für eine technische Umsetzung der Methodik bekannt. Dies ist darauf zurückzuführen, daß die beschriebenen Katalysatorsysteme häufig nur mit nicht ökonomischen Ausgangsmaterialien wie Iodaromaten befriedigende katalytische Wechselzahlen ("turnover numbers") ergeben. Ansonsten müssen bei Bromaromaten und insbesondere bei Chloraromaten generell große Mengen an Katalysator - allgemein 1-5 Mol-% - zugesetzt werden, um technisch nutzbare Umsätze zu erzielen. Aufgrund der Komplexität der Reaktionsgemische ist zudem kein einfaches Katalysatorrecycling möglich, so daß auch die Katalysatorkosten in der Regel einer technischen Realisierung entgegenstehen.

Daher bestand ein großer Bedarf nach einem Verfahren, das die genannten Nachteile nicht aufweist, für die technische Durchführung geeignet ist und aromatische Olefine in hoher Ausbeute und Reinheit liefert.

Diese Aufgabe wird gelöst durch ein Verfahren zur Herstellung von monofunktionellen, bi- und polyfunktionellen aromatischen Olefinen der Formel (I) 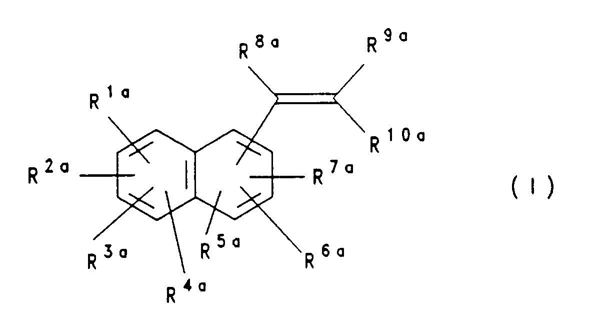
worin
R^{1a} bis R^{7a} unabhängig voneinander Wasserstoff, C₁-C₈-Alkyl, Alkoxy-(C₁-C₈), Acyloxy-(C₁-C₈), OPhenyl, Phenyl, Fluor, Chlor, Brom, Iod, OH, NO₂, OSO₂CF₃, CN, COOH, CHO, SO₃H, SO₂R, SOR, NH₂, NHAlkyl (C₁-C₈), N-Alkyl₂-(C₁-C₈), CHal₃, NHCO-Alkyl-(C₁-C₄), N-Alkyl-(C₁-C₄)-CO-Alkyl-(C₁-C₄), COO-Alkyl-(C₁-C₈), CONH₂, CO-Alkyl-(C₁-C₈), NHCOH, NCOO-Alkyl-(C₁-C₄), CO-Phenyl, COO-Phenyl, PO-Phenyl₂, PO-Alkyl₂-(C₁-C₄), wobei einer der Reste R^{1a} bis R^{7a} auch 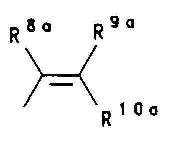
darstellen kann,
R^{8a} Wasserstoff, Alkyl(C₁-C₈), Phenyl, O-Alkyl-(C₁-C₈), Fluor R^{9a} und R^{10a} unabhängig voneinander Wasserstoff, CN, CO₂H, CO₂-Alkyl-(C₁-C₈), CONH₂, CONH-Alkyl-(C₁-C₄), CON(Alkyl)₂-(C₁-C₄), Fluor, CO₂-Phenyl, Alkyl, (C₁-C₈)-Phenyl, PO(Phenyl), PO(Alkyl-(C₁-C₄))₂, CO-Phenyl, CO-Alkyl-(C₁-C₄), O-Alkyl-(C₁-C₄), NH-Alkyl-(C₁-C₄), PO₃H, SO₃H, SO₃-Alkyl-(C₁-C₄), SO₂-Alkyl-(C₁-C₄), O-Phenyl, CHO,
bedeuten, durch Umsetzung von Halogenaromaten der allgemeinen Formel (II) 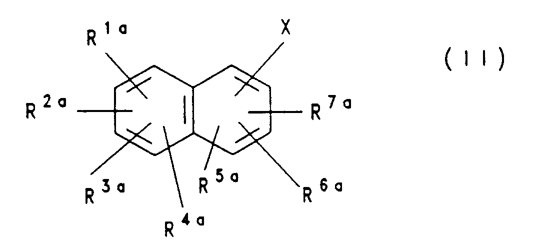
mit Olefinen der allgemeinen Formel (III) 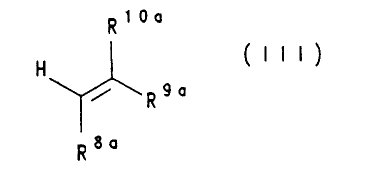
worin R^{1a} bis R^{10a} die angegebene Bedeutung besitzen, wobei einer der Reste R^{1a} bis R^{7a} auch für X stehen kann und X Iod, Brom, Chlor, OSO₂CF₃, OSO₂Phenyl, OSO₂CH₃ bedeutet, dadurch gekennzeichnet, daß man als Katalysator eine Palladiumverbindung der allgemeinen Formel (IV) 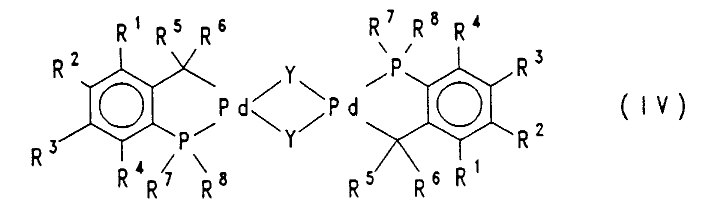
worin
R¹, R², R³, R⁴, R⁵, R⁶ unabhängig voneinander Wasserstoff, (C₁-C₄)-Alkyl, (C₁-C₄)-Alkoxy, Fluor, NH₂, NH-Alkyl(C₁-C₄), N(Alkyl)₂-(C₁-C₄), CO₂Alkyl-(C₁-C₄), OCO-Alkyl-(C₁-C₄), oder Phenyl bedeuten,
oder R¹ und R², R² und R³, R³ und R⁴, R⁵ und R⁶ zusammen einen aliphatischen oder aromatischen Ring bilden, und
R⁷, R⁸ (C₁-C₈)-Alkyl, (C₃-C₁₂)-Cycloalkyl, substituiertes oder unsubstituiertes Aryl sind
Y ein Anion einer anorganischen oder organischen Säure bedeutet, einsetzt.

In vielen Fällen haben sich Verbindungen der Formel (IV), worin R¹ bis R⁶ Wasserstoff, Alkyl(C₁-C₄), Phenyl, Cycloalkyl-(C₅-C₈), R⁷ und R⁸ Phenyl, Tolyl, Xylyl, Mesityl, Alkyl(C₁-C₈) und Cycloalkyl(C₅-C₈) bedeuten und Y für Acetat, Propionat, Benzoat, Chlorid, Bromid, Iodid, Fluorid, Sulfat, Hydrogensulfat, Nitrat, Phosphat, Tetrafluoroborat, Tosylat, Mesylat, Acetylacetonat, Hexafluoracetylacetonat oder Pyrazolyl steht, bewährt.

Gut geeignet sind z.B. Verbindungen wo R¹ - R⁶ für H, Alkyl, Phenyl und R⁷, R⁸ für Alkyl, Phenyl, Tolyl, Mesityl und Xylyl stehen.

Sehr gute Ergebnisse liefern die Verbindungen:
trans-Di-µ-acetato-bis[o-(di-o-tolylphosphino)benzyl]dipalladium(II)
trans-Di-µ-chloro-bis[o-(di-o-tolylphosphino)benzyl]dipalladium(II)
trans-Di-µ-bromo-bis[o-(di-o-tolylphosphino)benzyl]dipalladium(II)
trans-Di-µ-iodo-bis[o-(di-o-tolylphosphino)benzyl]dipalladium(II)
trans-Di-µ-acetato-bis[o-(dimesitylphosphino)-3,5-dimethylbenzyl]dipalladium(II)
trans-Di-µ-chloro-bis[o-(dimesitylphosphino)-3,5-dimethylbenzyl]dipalladium(II)
trans-Di-µ-bromo-bis[o-(dimesitylphosphino)-3,5-dimethylbenzyl]dipalladium(II)
trans-Di-µ-iodo-bis[o-(dimesitylphosphino)-3,5-dimethylbenzyl]dipalladium(II)
trans-Di-µ-acetato-bis[o-(t-butyl-o-tolylphosphino)benzyl]dipalladium(II)
trans-Di-µ-acetato-bis[o-(di-t-butylphosphino)benzyl]dipalladium(II)
trans-Di-µ-acetato-bis[o-(cyclohexyl-o-tolylphosphino)benzyl]dipalladium(II).

Das Verfahren hat sich insbesondere für die Herstellung von Verbindungen der Formel (I) bewährt, worin bedeuten:
R^{1a} bis R^{7a} unabhängig voneinander Wasserstoff, (C₁-C₈)-Alkyl, (C₁-C₈)-Alkoxy, (C₁-C₈)-Acyloxy, Phenyl, Fluor, Chlor, NO₂, CN, COOH, CHO, SO₂R, OH, NH-(C₁-C₈)-Alkyl, N[(C₁-C₈)Alkyl]₂, COO-(C₁-C₈)-Alkyl, CONH₂, CO-(C₁-C₈)-Alkyl, CO-Phenyl, COO-Phenyl, PO-(Phenyl)₂,
R^{8a} Wasserstoff, (C₁-C₈)-Alkyl,
R^{9a}, R^{10a} unabhängig voneinander Wasserstoff, CN, CO₂H, CO₂-(C₁-C₈)-Alkyl, CO₂-Phenyl, (C₁-C₈)-Alkyl, CO-Phenyl, CO-(C₁-C₄)-Alkyl.

Wichtig ist das Verfahren z.B. zur Herstellung von Verbindungen der Formel (I) worin bedeuten:
R^{1a} bis R^{7a} unabhängig voneinander Wasserstoff, (C₁-C₈)-Alkyl, (C₁-C₈)-Alkoxy, Phenyl, Fluor, Chlor, NO₂, CN, COOH, CHO, COO-(C₁-C₈)-Alkyl, CONH₂, OH, CO-(C₁-C₈)-Alkyl, CO-Phenyl, PO-(Phenyl)₂, R^{8a} Wasserstoff,
R^{9a}, R^{10a} unabhängig voneinander CN, CO₂H, CO₂-(C₁-C₈)-Alkyl, Wasserstoff, CO₂-Phenyl, CO-Phenyl, CO-(C₁-C₄)-Alkyl.

Besonderes Interesse findet das Verfahren zur Herstellung von Verbindungen der Formel (I) worin 4, insbesondere 5, bevorzugt 6 der Reste R^{1a} bis R^{7a} Wasserstoff bedeuten. Hierunter ist besonders die Synthese von Alkyloxyvinylnaphthalinen, Hydroxyvinylnaphthalinen, Acyloxyvinylnaphthalinen, 4-(Hydroxynaphthyl)-but-3-en-2-onen, 4-(Alkoxynaphthyl)-but-3-en-2-onen, 4-(Acyloxynaphthyl)-but-3-en-2-onen und insbesondere von 6-Methoxy-2-vinylnaphthalin und 4-(6-Methoxynaphthyl)-but-3-en-2-onen von großer technischer Bedeutung.

Als Lösungsmittel finden im allgemeinen inerte organische Lösungsmittel Verwendung. Gut geeignet sind dipolar aprotische Lösungsmittel wie Dialkylsulfoxide, N,N-Dialkylamide von aliphatischen Carbonsäuren oder alkylierte Lactame. Hierbei sind Dimethylsulfoxid, Dimethylacetamid, Dimethylformamid und N-Methylpyrrolidon bevorzugt.

Die Reaktion läuft bei Temperaturen von 20 bis 200°C ab, in vielen Fällen hat es sich bewährt, bei Temperaturen von 60 bis 180°C, bevorzugt 100 bis 150°C zu arbeiten.

Da bei der Reaktion HX abgespalten wird, ist es vorteilhaft, diese Säure durch Zusatz einer Base abzufangen. Dazu geeignet sind primäre, sekundäre oder tertiäre Amine wie Alkylamine, Dialkylamine, Trialkylamine, die alicyclisch oder offenkettig sein können und Alkali- oder Erdalkalisalze von aliphatischen oder aromatischen Carbonsäuren oder der Kohlensäure, wie Lithium, Natrium-, Kalium-, Calcium-, Magnesiumacetat und entsprechende Carbonate oder Hydrogencarbonate.

Die eingesetzten Palladiumkatalysatoren werden in der Regel vor der eigentlichen Reaktion isoliert synthetisiert, sie können jedoch auch in situ erzeugt werden, ohne daß dadurch die anfängliche katalytische Aktivität gemindert wird. Bei längerer Reaktionführung erweisen sich die in situ Mischungen (molares Verhältnis Pd:P = 1:1) jedoch als wenig stabil und führen häufig zur Palladiumabscheidung. Es muß daher bei in situ Mischungen mit einem Phosphan-Überschuß gearbeitet werden, welcher beim Einsatz der Palladacyclen entfällt.

Die Synthese der eingesetzten Palladiumkatalysatoren erfolgt gemäß dem Verfahren der deutschen Patentanmeldung P 44 21 753.

Die eingesetzten oder sich bildenden Palladacyclen haben in der Regel einen dimeren Aufbau. Bei bestimmten Verbindungen (z.B. Y = Acetylaceton, Hexafluoracetylaceton) können jedoch auch monomere, oligomere oder gar polymere Strukturen vorliegen.

Während des Katalysezyklus wird durch Brückenspaltungsreaktionen mit anorganischen und organischen Nucleophilen die dimere Struktur aufgebrochen, so daß als eigentlich katalytisch aktive Spezies die einkernigen Komplexe der Formel (V) bzw. (VI) 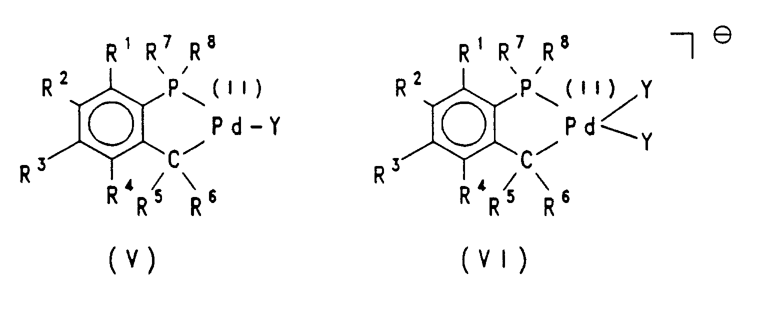
in Betracht zu ziehen sind. Die Komplexe der Formel (V) und (VI) stehen mit den tatsächlichen eingesetzten Dimeren im Austauschgleichgewicht und haben neutralen oder anionischen Charakter. Der einkernige Komplex der Formel (V) kann dabei gegebenenfalls weitere Donorliganden am Palladiumatom enthalten.

Der sehr vorteilhafte Verlauf der erfindungsgemäßen Reaktion war besonders überraschend, da nach dem Stand der Technik Palladiumkatalysatoren der Formel (IV) für die Durchführung der Heck-Reaktion als ungeeignet galten.

So stellt R.F. Heck ausdrücklich fest, daß Palladacyclen keine katalytische Aktivität für die Arylierung von Olefinen besitzen (T. Mitsudo, W. Fischetti, R.F. Heck, J. Org. Chem., Jg. 1984, Bd. 49, 1640).

Auch A.L. Rheingold und W.C. Fultz (Organometallics Jg. 1984, Bd. 3, 1414) beschreiben, daß sich bei der Heck-Reaktion von lodaromaten mit Dienen in Gegenwart von Palladiumacetat und Tris-(o-tolyl)phosphan Palladacyclen bilden, die keine katalytische Aktivität aufweisen.

Vor diesem Hintergrund sind die Vorteile der bei den erfindungsgemäßen Verfahren eingesetzten Katalysatoren höchst unerwartet und besonders überraschend.

Die als neue Katalysatorsysteme eingesetzten Palladacyclen zeichnen sich durch sehr große Aktivität und unerwarteterweise damit einhergehende hohe Stabilität aus.

Die Stabilität der Palladacyclen in Lösung läßt sich durch Zusatz von Alkali-, Erdalkali- und Übergangsmetallsalzen der 6. bis 8. Nebengruppe erhöhen. Insbesondere der Zusatz von Halogeniden und Pseudohalogeniden (z.B. CN⁻) bewirken bei der Umsetzung von Chloraromaten signifikante Ausbeutesteigerungen (1 bis 100 %) und Standzeitverbesserungen des Homogenkatalysators. Geeignet sind auch Tri- und Tetraalkylammonium-Salze sowie entsprechende Phosphonium- und Arsonium-Salze.

So können Turnover-Werte in der Größenordnung von 100.000 und mehr realisiert werden.

Aufgrund der Katalysatoraktivitäten und -stabilität ist es somit bei bestimmten Verfahren möglich, extrem kleine Mengen an Katalysator zu verwenden, so daß die Katalysatorkosten im Vergleich zu herkömmlichen Heck-Reaktionen für den entsprechenden Prozeß nicht kostenlimitierend sind.

Außerdem bedingt der Einsatz minimalster Mengen an Katalysator ökologische Vorteile, da Abfallprodukte oder abfallproduktträchtige Aufarbeitungsverfahren vermieden werden.

Die nachstehenden Beispiele dienen zur Erläuterung des erfindungsgemäßen Verfahrens, ohne es darauf zu beschränken.

### Beispiel 1: Synthese des Katalysators

### 1. trans-Di-(µ-acetato-bis[o-(di-o-tolylphosphino)benzyl]dipalladium(II) (1)

4,5 g (20 mmol] Pd(OAc)₂ werden in 500 ml Toluol mit rotbrauner Farbe gelöst. Die Lösung wird mit 8,0 g (26,3 mmol) Tri-(o-tolyl)phosphan versetzt. Die sich rasch nach hellorange aufklarende Lösung wird 3 Minuten lang auf knapp 50°C erhitzt und dann auf Raumtemperatur abgekühlt. Das Lösungsmittel wird im Vakuum bis auf 1/4 des Volumens entfernt. Nach Zugabe von 500 ml Hexan wird der ausgefallene Niederschlag abfiltriert. Man erhält 8,8 g (93 % d. Th.) bezogen auf Pd(OAc)₂) (1) als gelben Feststoff (Schmp. > 200°C). Durch Umkristallisation aus Toluol/Hexan oder Methylenchlorid/Hexan und Filtration der Lösungen über Celite® kann (1) in Form gelber Kristallnadeln analysenrein gewonnen werden.

### Elementaranalyse:

gef.: C, 58,89 %; H, 5,06 %; P, 6,92 %; O, 6,47 %; Pd, 21,84 %;
C₄₆H₄₆O₄P₂Pd₂ (937,62)
ber.: C, 58,93 %; H, 4,94 %; P, 6,61 %; O, 6,83 %; Pd, 22,70 %;
IR (cm⁻¹, KBr): 3052m, 3007m, 2954w, 2925m *v*(CH); 1578vs *v*(µ₂-C=O), 1468s; 1630 *v*(C=C); 1578, 1416 *v*/µ₂-CO); 1341;
¹H-NMR (400 MHz, -70°C, CD₂Cl₂): δ = 7.31 (4H, m, H_{Tolyl}); 7.21 (2H, m, H_{Tolyl}); 7.12 (6H, m, H_{Tolyl}); 7.06 (2H, t, H_{Benzyl}, ³J(HH) = 7.3 Hz); 6.92 (4H, m, H_{Tolyl}); 6.70 (2H, t, H_{Benzyl}, ³J(HH) = 7.3 Hz); 6.56 (2H, t, H_{Benzyl}, ³J(HH) = 9 Hz); 6.35 (2H, dd, H_{Benzyl}, ³J(HH) = 7.9 Hz, ⁴J(PH) = 12.2 Hz); 3.00 (6H, s, CH₃); 2.81 (2H, dd, CHₐH_{b}, ²J(HₐH_{b}) = 14.0 Hz, ³J(PH) = 4.3 Hz); 2.40 (2H, dd, CHₐH_{b}, ²J(HₐH_{b}) = 14.0 Hz, ³J(PH) = 1.8 Hz); 2.10 (6H, s, CH₃); 1.91 (s, 6H, CH₃);
¹³C{¹H}-NMR (100.5 MHz, -70°C, CD₂Cl₂): δ = 178.5 (s, CH₃CO₂); 157.1 (d, C_{Ar}, J(PC) = 31.3 Hz); 141.1 (d, C_{Ar}, J(PC) = 16.0 Hz); 141,0 (d, C_{Ar}, J(PC) = 21.0 Hz); 133.0 (s, C_{Ar}); 132.5 (d, C_{Ar}, J(PC) = 4.6 Hz); 132.4 (d, C_{Ar}, J(PC) = 6.1 Hz); 131.7 (d, C_{Ar}, J(PC) = 8.8 Hz); 131.4 (d, C_{Ar}; J(PC) = 13.7); 131.3 (d, C_{Ar}, J(PC) = 9.9 Hz); 130.4 (d, C_{Ar}, J(PC) = 16.0 Hz); 129.9 (s, C_{Ar}); 129.1 (d, C_{Ar}, J(PC) = 46.2 Hz); 128.7 (s, C_{Ar}); 128.1 (d, C_{Ar}, J(PC) = 33.2 Hz); 127.6 (d, C_{Ar}, J(PC) = 23.7 HZ); 125.6 (d, C_{Ar}, J(PC) = 7.3 Hz); 125.2 (d, C_{Ar}, J(PC) = 7.3 Hz); 124.9 (d. C_{Ar}, J(PC) = 11.4 Hz); 30.8 (s, CH₂); 24.7 (d, CH₃CO₂, 4J(PC) = 3.1 Hz);23.0 (d, CH₃, 3J(PC) = 13.7 Hz); 22.2 (d, CH₃, 3J(PC) = 6.9 Hz);
³¹P{¹H}-NMR (161.9 MHz, -70°C, CD₂Cl₂): δ = 34.2 (s);
CI-MS (150 eV): m/e = 939 [M⁺ +H], 880 [M⁺-OAc], 819 [M⁺-2OAc], 714 [Pd{o-CH₂C₆H₄P(o-Tol)₂}₂⁺].

### Beispiel 2: Darstellung von 2-Vinyl-6-methoxynaphthalin

23,7 g 2-Brom-6-methoxynaphthalin (100 mmol) werden in 150 ml N,N-Dimethylacetamid gelöst. Es wird 1 mg 2,6-Di-tert.-butylphenol, 10 g NaOAc (1,2 eq) und 50 mg Palladacyclus zugesetzt. Die Lösung wird in einen V4A-Rührautoklav überführt und bei 140°C und einem Ethylendruck von 20 bar bis zum vollständigen Umsatz gerührt.
Zeit: 10 bis 16 Stunden
Umsatz: > 95 % (GC)
Ausbeute: 89 %
Aufarbeitung: Nach dem Abkühlen auf RT wird der Feststoff abfiltriert und mit 20 ml DMAc nachgewaschen. Mit 200 bis 400 ml 5 % HCl wird aus der Mutterlauge das Produkt gefällt. Die Lösung ist während der HCl-Zugabe bei Raumtemperatur zu halten. Der ausgefallene Feststoff wird portionsweise mit MeOH gewaschen und am HV getrocknet.

### Beispiel 3: Darstellung von 2-Vinyl-6-methoxynaphthalin

Durchführung analog Beispiel 2.
Druck: 30 bar Ethylen
Umsatz: 87 %
Ausbeute: 80 %

### Beispiel 4: Darstellung von 4-(6-Methoxy-naphthyl)-but-3-en-2-on Reaktion unter Schutzgas

6,0 g 2-Brom-6-methoxynaphthalin (25,3 mmol) werden in 25 ml N,N-Dimethylacetamid gelöst. Es wird 1 mg 2,6-Di-tert.-butylphenol, 3,2 g NaOAc (1,2 eq) und 1,2 mg Palladacyclus (0,01 mol%) zugesetzt. Nach der Zugabe von 3,1 ml Methylvinylketon (1,5 eq) wird 6 bis 8 Stunden bei 140°C gerührt.
Umsatz: 100 % (GC)
Aufarbeitung: Nach dem Abkühlen auf RT wird der Feststoff abfiltriert und mit 20 bis 40 ml Dichlormethan nachgewaschen. Die Mutterlauge wird 3 mal mit 20 ml Wasser extrahiert, anschließend wird die organische Phase am RV eingeengt. Nach Säulenchromatographie erhält man eine Ausbeute von 87 %.

### Beispiel 5: Darstellung von 4-(6-Methoxynaphthyl)-but-3-en-2-on Reaktion unter Schutzgas

Durchführung analog Beispiel 4. Als Base wurde 1,2 eq Natriumcarbonat eingesetzt.
Umsatz 100 % (GC)
Aufarbeitung wie oben. Es wurde aus MeOH umkristallisiert.
Erhaltene Ausbeute: 82 %.

## Patentansprüche

1. Verfahren zur Herstellung von monofunktionellen, bi- und polyfunktionellen aromatischen Olefinen der Formel (I) 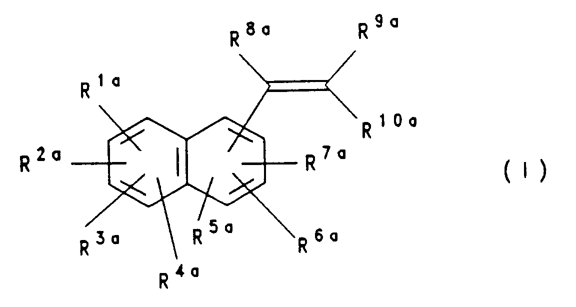 worin
R^{1a} bis R^{7a} unabhängig voneinander Wasserstoff, C₁-C₈-Alkyl, Alkoxy-(C₁-C₈), Acyloxy-(C₁-C₈), OPhenyl, Phenyl, Fluor, Chlor, Brom, Iod, OH, NO₂, OSO₂CF₃, CN, COOH, CHO, SO₃H, SO₂R, SOR, NH₂, NHAlkyl (C₁-C₈), N-Alkyl₂-(C₁-C₈), CHal₃, NHCO-Alkyl-(C₁-C₄), N-Alkyl-(C₁-C₄)-CO-Alkyl-(C₁-C₄), COO-Alkyl-(C₁-C₈), CONH₂, CO-Alkyl-(C₁-C₈), NHCOH, NCOO-Alkyl-(C₁-C₄), CO-Phenyl, COO-Phenyl, PO-Phenyl₂, PO-Alkyl₂-(C₁-C₄), wobei einer der Reste R^{1a} bis R^{7a} auch 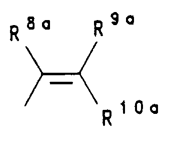 darstellen kann,
R^{8a} Wasserstoff, Alkyl(C₁-C₈), Phenyl, O-Alkyl-(C₁-C₈), Fluor R^{9a} und R^{10a} unabhängig voneinander Wasserstoff, CN, CO₂H, CO₂-Alkyl-(C₁-C₈), CONH₂, CONH-Alkyl-(C₁-C₄), CON(Alkyl)₂-(C₁-C₄), Fluor, CO₂-Phenyl, Alkyl, (C₁-C₈)-Phenyl, PO(Phenyl), PO(Alkyl-(C₁-C₄))₂, CO-Phenyl, CO-Alkyl-(C₁-C₄), O-Alkyl-(C₁-C₄), NH-Alkyl-(C₁-C₄), PO₃H, SO₃H, SO₃-Alkyl-(C₁-C₄), SO₂-Alkyl-(C₁-C₄), O-Phenyl, bedeuten, durch Umsetzung von Halogenaromaten der allgemeinen Formel (II) 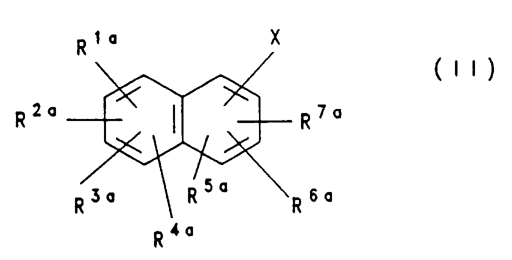 mit Olefinen der allgemeinen Formel (III) 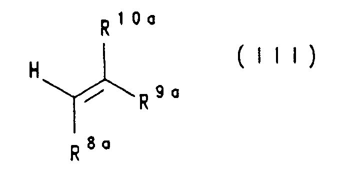 worin R^{1a} bis R^{10a} die angegebene Bedeutung besitzen, wobei einer der Reste R^{1a} bis R^{7a} auch für X stehen kann und X Iod, Brom, Chlor, OSO₂CF₃, OSO₂Phenyl, OSO₂CH₃ bedeutet, dadurch gekennzeichnet, daß man als Katalysator eine Palladiumverbindung der allgemeinen Formel (IV) 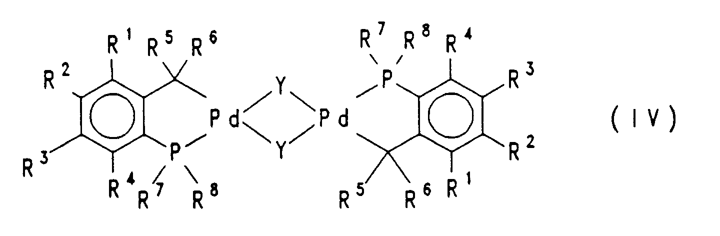 worin R¹, R², R³, R⁴, R⁵, R⁶ unabhängig voneinander Wasserstoff, (C₁-C₄)-Alkyl, (C₁-C₄)-Alkoxy, Fluor, NH₂, NH-Alkyl(C₁-C₄), N(Alkyl)₂-(C₁-C₄), CO₂Alkyl-(C₁-C₄), OCO-Alkyl-(C₁-C₄), oder Phenyl bedeuten, oder R¹ und R², R² und R³, R³ und R⁴, R⁵ und R⁶ zusammen einen aliphatischen oder aromatischen Ring bilden, und
R⁷, R⁸ (C₁-C₈)-Alkyl, (C₃-C₁₂)-Cycloalkyl, substituiertes oder unsubstituiertes Aryl sind
Y ein Anion einer anorganischen oder organischen Säure bedeutet, einsetzt.

2. Verfahren nach Anspruch 1, dadurch gekennzeichnet, daß in Formel (IV) R¹ bis R⁶ unabhängig voneinander Wasserstoff, (C₁-C₄)-Alkyl, (C₅-C₈)-Cycloalkyl,
R⁷, R⁸ Phenyl, Tolyl, Xylyl, Mesityl, Alkyl-(C₁-C₈), Cycloalkyl-(C₅-C₈) bedeuten und
Y für Acetat, Propionat, Benzoat, Chlorid, Bromid, Iodid, Fluorid, Sulfat, Hydrogensulfat, Nitrat, Phosphat, Tetrafluoroborat, Tosylat, Mesylat, Acetylacetonat, Hexafluoracetylacetonat oder Pyrazolyl steht.

3. Verfahren nach Anspruch 1, dadurch gekennzeichnet, daß als Katalysator die Verbindungen
trans-Di-µ-acetato-bis[o-(di-o-tolylphosphino)benzyl]dipalladium(II)
trans-Di-µ-chloro-bis[o-(di-o-tolylphosphino)benzyl]dipalladium(II)
trans-Di-µ-bromo-bis[o-(di-o-tolylphosphino)benzyl]dipalladium(II)
trans-Di-µ-iodo-bis[o-(di-o-tolylphosphino)benzyl]dipalladium(II)
trans-Di-µ-acetato-bis(o-(dimesitylphosphino)-3,5-dimethylbenzyl]dipalladium(II)
trans-Di-µ-chloro-bis[o-(dimesitylphosphino)-3,5-dimethylbenzyl]dipalladium(II)
trans-Di-µ-bromo-bis[o-(dimesitylphosphino)-3,5-dimethylbenzyl]dipalladium(II)
trans-Di-µ-iodo-bis[o-(dimesitylphosphino)-3,5-dimethylbenzyl]dipalladium(II)
trans-Di-µ-acetato-bis[o-(t-butyl-o-tolylphosphino)benzyl]dipalladium(II)
trans-Di-µ-acetato-bis[o-(di-t-butylphosphino)benzyl]dipalladium(II)
trans-Di-µ-acetato-bis[o-(cyclohexyl-o-tolylphosphino)benzyl]dipalladium(II)
eingesetzt werden.

4. Verfahren nach mindestens einem der Ansprüche 1 bis 3, dadurch gekennzeichnet, daß der Katalysator in situ hergestellt wird.

5. Verfahren nach mindestens einem der Ansprüche 1 bis 4, dadurch gekennzeichnet, daß in Formel (I) bedeuten:
R^{1a} bis R^{7a} unabhängig voneinander Wasserstoff, (C₁-C₈)-Alkyl, (C₁-C₈)-Alkoxy, (C₁-C₈)-Acyloxy, Phenyl, Fluor, Chlor, NO₂, CN, COOH, CHO, SO₂R, NH-(C₁-C₈)-Alkyl, N[(C₁-C₈)Alkyl]₂, COO-(C₁-C₈)-Alkyl, CONH₂, CO-(C₁-C₈)-Alkyl, CO-Phenyl, COO-Phenyl, PO-(Phenyl)₂, OH, R^{8a} Wasserstoff, (C₁-C₈)-Alkyl,
R^{9a}, R^{10a} unabhängig voneinander Wasserstoff, CN, CO₂H, CO₂-(C₁-C₈)-Alkyl, CO₂-Phenyl, (C₁-C₈)-Alkyl, CO-Phenyl, CO-(C₁-C₄)-Alkyl.

6. Verfahren nach mindestens einem der Ansprüche 1 bis 4, dadurch gekennzeichnet, daß in Formel (I) bedeuten:
R^{1a} bis R^{7a} unabhängig voneinander Wasserstoff, (C₁-C₈)-Alkyl, (C₁-C₈)-Alkoxy, Phenyl, Fluor, Chlor, NO₂, CN, COOH, CHO, COO-(C₁-C₈)-Alkyl, CONH₂, CO-(C₁-C₈)-Alkyl, CO-Phenyl, PO-(Phenyl)₂, OH, R^{8a} Wasserstoff,
R^{9a}, R^{10a} unabhängig voneinander CN, CO₂H, CO₂-(C₁-C₈)-Alkyl, Wasserstoff, CO₂-Phenyl, CO-Phenyl, CO-(C₁-C₄)-Alkyl.

7. Verfahren nach mindestens einem der Ansprüche 1 bis 6, dadurch gekennzeichnet, daß in Formel (I) 4, insbesondere 5, bevorzugt 6 der Reste R^{1a} bis R^{7a} Wasserstoff bedeuten.

8. Verfahren nach mindestens einem der Ansprüche 1 bis 4, dadurch gekennzeichnet, daß Formel (I) für Alkyloxyvinylnaphthaline, Hydroxyvinylnaphthaline, Acyloxyvinylnaphthaline, 4-(Hydroxynaphthyl)-but-3-en-2-one, 4-(Alkoxynaphthyl)-but-3-en-2-one, 4-(Acyloxynaphthyl)-but-3-en-2-one und insbesondere von 6-Methoxy-2-vinylnaphthalin und 4-(6-Methoxynaphthyl)-but-3-en-2-on steht.

9. Verfahren nach mindestens einem der Ansprüche 1 bis 8, dadurch gekennzeichnet, daß als Lösungsmittel dipolar aprotische Lösungsmittel, insbesondere Dialkylsulfoxide, N,N-Dialkylamide, alkylierte Lactame, bevorzugt Diemthylsulfoxid, Dimethylacetamid, Dimethylformamid oder N-Methylpyrrolidon eingesetzt werden.

10. Verfahren nach mindestens einem der Ansprüche 1 bis 9, dadurch gekennzeichnet, daß die Reaktion bei Temperaturen von 20 bis 200°C, insbesondere 60 bis 180°C, bevorzugt 100 bis 150°C durchgeführt wird.

11. Verfahren nach mindestens einem der Ansprüche 1 bis 10, dadurch gekennzeichnet, daß die bei der Reaktion entstehende Säure HX durch Zusatz einer Base, insbesondere eines Amins oder eines Alkali oder Erdalkalimetallsalzes einer schwachen Säure abgefangen wird.

12. Verfahren nach Anspruch 11, dadurch gekennzeichnet, daß als Base Alkylamine oder die Carbonate, Hydrogencarbonate oder Acetate von Lithium, Natrium, Kalium, Calcium oder Magnesium eingesetzt werden.

13. Verfahren nach mindestens einem der Ansprüche 1 bis 12, dadurch gekennzeichnet, daß Salze von Halogeniden und Pseudohalogeniden der Alkali-, Erdalkalimetalle und Metalle der 6. bis 8. Nebengruppe zugesetzt werden.

14. Verfahren nach mindestens einem der Ansprüche 1 bis 13, dadurch gekennzeichnet, daß Tri- oder Tetraalkylammonium, -phosphonium oder -arsoniumsalze zugesetzt werden.
